# DEMANDE DE BREVET EUROPEEN

(11) **EP 2 356 975 A1**
(43) Date de publication de la demande: **17.08.2011**
(21) Numéro de dépôt: 10196182.9
(22) Date de dépôt: 21.12.2010
(51) Int. Cl.: A61K 8/31, A61K 8/34, A61K 8/37, A61K 8/73, A61K 8/81, A61K 8/891, A61Q 5/06, A61Q 5/12

(54) **Composition cosmétique comprenant au moins deux alcanes linéaires volatiles, et au moins un polymère cationique non-protéique**

(30) Priorité: 23.12.2009 FR 0959482
(71) Demandeur: L'Oréal, 75008 Paris (FR)
(72) Inventeur: Desenne, Patricia, 74370, Pringy (FR); Bourdin, Claire, 92300, Levallois Perret (FR)
(74) Mandataire: Dossmann, Gérard

(57) **Abrégé**

La présente invention concerne une composition cosmétique comprenant dans un milieu cosmétiquement acceptable :
- deux ou plus de deux alcanes linéaires volatils, et
- un ou plusieurs polymères cationiques non-protéiques,

avec un rapport pondéral alcanes linéaires volatils/polymère(s) supérieur ou égal à 1,2.

Elle concerne aussi son utilisation pour le traitement cosmétique des matières kératiniques, de préférence des fibres kératiniques comme les cheveux, et un procédé de traitement cosmétique des matières kératiniques mettant en oeuvre ladite composition.

## Description

La présente invention concerne une composition cosmétique comprenant au moins deux alcanes linéaires volatils et au moins un polymère cationique non-protéique, son utilisation pour le traitement cosmétique des matières kératiniques, de préférence des fibres kératiniques comme les cheveux, et un procédé de traitement cosmétique des matières kératiniques mettant en oeuvre ladite composition.

Dans le domaine du traitement capillaire, l'utilisation de solvants volatils est connue dans des produits capillaires de soins rincés ou non rincés. Ils sont généralement utilisés pour différentes raisons. Ils permettent notamment de modifier le rendu sensoriel d'un produit capillaire en lui conférant une texture légère et non collante dans la main. Ils peuvent également lui conférer un caractère glissant qui facilite la répartition du produit sur les cheveux et en particulier sur des cheveux secs.

Dans les émulsions aqueuses de type huile-dans-eau, qui peuvent se présenter sous forme de crèmes plus ou moins gélifiées, l'ajout de solvants volatils peut également permettre de solubiliser des gommes de silicone qui, de par leur viscosité intrinsèque, seraient difficiles à introduire dans les compositions.

Ces solvants volatils qui sont généralement des esters gras liquides, des huiles hydrocarbonées de type isododécane ou isohexadécane, et/ou des huiles siliconées, peuvent notamment conduire à des problèmes de toucher gras, de manque de brillance, et de cheveux raidis et durs.

Il subsiste donc un besoin de remplacer ces solvants volatils pour éviter les inconvénients cités ci-dessus.

La demanderesse a maintenant découvert de façon inattendue et surprenante, que l'association d'au moins deux alcanes linéaires volatils et d'au moins un polymère cationique non-protéique permettait d'éviter les inconvénients mentionnés ci-dessus et d'améliorer les propriétés cosmétiques telles que le lissage, la brillance, la transformation de la fibre au rinçage, le démêlage, la légèreté, la souplesse, la douceur, l'effet corporisant, et la définition des boucles sur cheveux bouclés.

Par « transformation de la fibre au rinçage », on entend une fibre immédiatement assouplie, douce et lisse au moment où l'on rince le produit de soin rincé.

On obtient un « effet corporisant » lorsque l'on sent dans la main un effet de masse des cheveux et une sensation de densité globale de la chevelure.

En particulier, l'application sur cheveux humides permet d'obtenir des cheveux humides plus lisses et plus brillants. En outre, les cheveux secs sont lisses et soignés sans alourdissement.

Ainsi, l'invention a pour objet une composition cosmétique comprenant, dans un milieu cosmétiquement acceptable, deux ou plus de deux alcanes linéaires volatils et un, deux ou plus de deux polymères cationiques non-protéiques en un rapport pondéral alcanes linéaires volatils/polymère(s) cationique(s) non-protéique(s) supérieur ou égal à 1,2.

Elle a encore pour objet l'utilisation de ladite composition pour le traitement cosmétique des matières kératiniques, de préférence des fibres kératiniques comme les cheveux.

Un autre objet de l'invention est un procédé de traitement cosmétique des matières kératiniques, de préférence des fibres kératiniques comme les cheveux, mettant en oeuvre ladite composition.

La composition cosmétique selon l'invention comprend, dans un milieu cosmétiquement acceptable :
- deux ou plus de deux alcanes linéaires volatils et
- un ou plusieurs polymères cationiques non-protéiques
en un rapport pondéral alcanes linéaires volatils/polymère(s) cationique(s) non-protéique(s) supérieur ou égal à 1,2.

Selon un mode de réalisation, la composition cosmétique comprend, dans un milieu cosmétiquement acceptable :
- deux ou plus de deux alcanes linéaires en C_{7-15,} de préférence en C₈₋₁₄, et
- un ou plusieurs polymères cationiques non-protéiques
en un rapport pondéral alcanes linéaires volatils/polymère(s) cationique(s) non-protéique(s) supérieur ou égal à 1,2.

Ledit rapport va de préférence de 1,2 à 50, mieux de 2 à 30 et mieux encore de 3 à 20.

Par « deux ou plus de deux alcanes linéaires volatils», on entend indifféremment « deux ou plus de deux huiles alcanes linéaires volatiles ».

Un alcane linéaire volatil convenant à l'invention est liquide à température ambiante (environ 25 °C) et sous pression atmosphérique (101 325 Pa ou 760 mm Hg).

Par « alcane linéaire volatil » convenant à l'invention, on entend un alcane linéaire, susceptible de s'évaporer au contact de la peau en moins d'une heure, à température ambiante (25°C) et sous pression atmosphérique (101 325 Pa), liquide à température ambiante, ayant notamment une vitesse d'évaporation allant de 0,01 à 15 mg/cm²/min, à température ambiante (25°C) et sous pression atmosphérique (101 325 Pa).

De préférence, les alcanes linéaires volatils convenant à l'invention présentent une vitesse d'évaporation allant de 0,01 à 3,5 mg/cm²/min, mieux de 0,01 à 1,5 mg/cm²/min, à température ambiante (25°C) et sous pression atmosphérique (101 325 Pa).

De façon plus préférée, les alcanes linéaires volatils convenant à l'invention présentent une vitesse d'évaporation allant de 0,01 à 0,8 mg/cm²/min, préférentiellement de 0,01 à 0,3 mg/cm²/min, et encore plus préférentiellement de 0,01 à 0,12 mg/cm²/min, à température ambiante (25°C) et sous pression atmosphérique (101 325 Pa).

La vitesse d'évaporation d'un alcane volatil conforme à l'invention (et plus généralement d'un solvant volatil) peut être notamment évaluée au moyen du protocole décrit dans WO 06/013413, et plus particulièrement au moyen du protocole décrit ci-après.

On introduit dans un cristallisoir (diamètre : 7 cm) placé sur une balance se trouvant dans une enceinte d'environ 0,3 m³ régulée en température (25 °C) et en hygrométrie (humidité relative 50 %), 15 g de solvant hydrocarboné volatil.

On laisse le solvant hydrocarboné volatil s'évaporer librement, sans l'agiter, en assurant une ventilation par un ventilateur (PAPST-MOTOREN, référence 8550 N, tournant à 2700 tours/minute) disposé en position verticale au-dessus du cristallisoir contenant le solvant hydrocarboné volatil, les pales étant dirigées vers le cristallisoir, à une distance de 20 cm par rapport au fond du cristallisoir.

On mesure à intervalles de temps réguliers la masse de solvant hydrocarboné volatil restante dans le cristallisoir.

On obtient alors le profil d'évaporation du solvant en traçant la courbe de la quantité de produit évaporé (en mg/cm²) en fonction du temps (en min).

Puis on calcule la vitesse d'évaporation qui correspond à la tangente à l'origine de la courbe obtenue. Les vitesses d'évaporation sont exprimées en mg de solvant volatil évaporé par unité de surface (cm²) et par unité de temps (minute).

Selon un mode de réalisation préféré, les alcanes linéaires volatils convenant à l'invention ont une pression de vapeur (appelée également pression de vapeur saturante) non nulle, à température ambiante, en particulier une pression de vapeur allant de 0,3 Pa à 6000 Pa.

De façon préférée, les alcanes linéaires volatils convenant à l'invention ont une pression de vapeur allant de 0,3 à 2000 Pa, mieux de 0,3 à 1000 Pa, à température ambiante (25°C).

De façon plus préférée, les alcanes linéaires volatils convenant à l'invention ont une pression de vapeur allant de 0,4 à 600 Pa, préférentiellement de 1 à 200 Pa, et encore plus préférentiellement de 3 à 60 Pa, à température ambiante (25°C).

Selon un mode de réalisation, un alcane linéaire volatil convenant à l'invention peut présenter un point éclair compris dans l'intervalle variant de 30 à 120 °C, et plus particulièrement de 40 à 100 °C. Le point éclair est en particulier mesuré selon la Norme iso 3679.

Selon un mode de réalisation, les alcanes linéaires volatils convenant dans l'invention peuvent comprendre de 7 à 15 atomes de carbone, de préférence de 8 à 14 atomes de carbone et mieux de 9 à 14 atomes de carbone.

De façon plus préférée, les alcanes linéaires volatils convenant dans l'invention comprennent de 10 à 14 atomes de carbone, et encore plus préférentiellement de 11 à 14 atomes de carbone.

Un alcane linéaire volatil convenant à l'invention peut être avantageusement d'origine végétale.

De préférence, le mélange d'alcanes linéaires volatils présent dans la composition selon l'invention comprend au moins un isotope ¹⁴C du carbone (carbone 14). En particulier, l'isotope ¹⁴C peut être présent en un rapport en nombre d'isotopes (ou ratio ¹⁴C/¹²C) supérieur ou égal à 1.10⁻¹⁶, de préférence supérieur ou égal à 1.10⁻¹⁵, de préférence encore supérieur ou égal 7,5.10⁻¹⁴, et mieux supérieur ou égal 1,5.10⁻¹³. De préférence, le ratio ¹⁴C / ¹²C va de 6.10⁻¹³ à 1,2.10⁻¹²

La quantité d'isotopes ¹⁴C dans le mélange d'alcanes linéaires volatils peut être déterminée par des méthodes connues de l'homme du métier telles que la méthode de comptage de Libby, la spectrométrie à scintillation liquide ou encore la spectrométrie de masse à accélération (Accelerator Mass Spectrometry).

Un tel alcane peut être obtenu, directement ou en plusieurs étapes, à partir d'une matière première végétale comme une huile, un beurre, une cire, etc.

A titre d'exemples d'alcanes convenant à l'invention, on peut mentionner les alcanes décrits dans les demandes de brevets WO 2007/068371 et WO2008/155059. Ces alcanes sont obtenus à partir d'alcools gras, eux-mêmes obtenus à partir d'huile de coprah ou de palme.

A titre d'exemples d'alcanes linéaires convenant à l'invention, on peut citer le n- heptane (C7), le n-octane (C8), le n-nonane (C9), le n-décane (C10), le n-undécane (C11), le n-dodécane (C12), le n-tridécane (C13), le n-tétradécane (C14), le n-pentadécane (C15). Selon un mode de réalisation particulier, les alcanes linéaires volatils sont choisis parmi le n-nonane, le n-undécane, le n-dodécane, le n-tridécane, le n-tétradécane.

Selon un mode de réalisation préféré, on peut citer les mélanges de n-undécane (C11) et de n-tridécane (C13) obtenus aux exemples 1 et 2 de la demande WO2008/155059.

On peut également citer le n-dodécane (C12) et le n-tétradécane (C 14) vendus respectivement sous les références PARAFOL 12-97 et PARAFOL 14-97 par la société Sasol, ainsi que leurs mélanges.

Un mode de réalisation consiste à utiliser un mélange d'au moins deux alcanes linéaires volatils distincts, différant entre eux d'un nombre de carbone n d'au moins 1, en particulier différant entre eux d'un nombre de carbone de 1 ou de 2.

Selon un mode de réalisation, on utilise un mélange d'au moins deux alcanes linéaires volatils distincts comportant de 10 à 14 atomes de carbone et différant entre eux d'un nombre de carbone d'au moins 1. A titre d'exemples, on peut citer notamment les mélanges d'alcanes linéaires volatils C10/C11, C11/C12, ou C12/C13.

Selon un autre mode de réalisation, on utilise un mélange d'au moins deux alcanes linéaires volatils distincts comportant de 10 à 14 atomes de carbone et différant entre eux d'un nombre de carbone d'au moins 2. A titre d'exemples, on peut citer notamment les mélanges d'alcanes linéaires volatils C10/C12, ou C12/C14, pour un nombre de carbone n pair et le mélange C11/C13 pour un nombre de carbone n impair.

Selon un mode de réalisation préféré, on utilise un mélange d'au moins deux alcanes linéaires volatils comportant de 10 à 14 atomes de carbone distincts et différant entre eux d'un nombre de carbone d'au moins 2, et en particulier un mélange d'alcanes linéaires volatils C11/C13 ou un mélange d'alcanes linéaires volatils C12/C14.

D'autres mélanges associant plus de 2 alcanes linéaires volatils selon l'invention, tels que, par exemple, un mélange d'au moins 3 alcanes linéaires volatils comportant de 7 à 15 atomes de carbone distincts et différant entre eux d'un nombre de carbone d'au moins 1, peuvent être utilisés dans l'invention.

Dans le cas des mélanges de deux alcanes linéaires volatils, lesdits deux alcanes linéaires volatils représentent de préférence plus de 95% et mieux plus de 99% en poids du mélange.

Selon un mode particulier de l'invention, dans un mélange d'alcanes linéaires volatils, l'alcane linéaire volatil ayant le nombre de carbone le plus petit est majoritaire dans le mélange.

Selon un autre mode de l'invention, on utilise un mélange d'alcanes linéaires volatils dans lequel l'alcane linéaire volatil ayant le nombre de carbone le plus grand est majoritaire dans le mélange.

A titre d'exemples de mélanges convenant à l'invention, on peut citer notamment les mélanges suivants :
- de 50 à 90% en poids, de préférence de 55 à 80% en poids, préférentiellement encore de 60 à 75% en poids d'alcane linéaire volatil en Cₙ avec n allant de 7 à 15
- de 10 à 50% en poids, de préférence de 20 à 45% en poids, de préférence de 24 à 40% en poids, d'alcane linéaire volatil en Cₙ₊ₓ avec x supérieur ou égal à 1, de préférence x=1 ou x=2, avec n+x compris entre 8 et 14,
par rapport au poids total des alcanes dans ledit mélange.

En particulier, ledit mélange d'alcanes linéaires volatils peut contenir en outre :
- moins de 2% en poids, de préférence moins de 1% en poids d'hydrocarbures ramifiés,
- et/ou moins de 2% en poids, de préférence moins de 1% en poids d'hydrocarbures aromatiques,
- et/ou moins de 2% en poids, de préférence moins de 1% en poids et préférentiellement moins de 0,1% en poids d'hydrocarbures insaturés,
   lesdits pourcentages étant exprimés par rapport au poids total du mélange.

Plus particulièrement, les alcanes linéaires volatils convenant dans l'invention peuvent être mis en oeuvre sous la forme d'un mélange n-undécane/n-tridécane.

En particulier, on utilisera un mélange d'alcanes linéaires volatils comprenant :
- de 55 à 80% en poids, de préférence de 60 à 75% en poids d'alcane linéaire volatil en C11 (n-undécane) et
- de 20 à 45% en poids, de préférence de 24 à 40% en poids d'alcane linéaire volatil en C13 (n-tridécane),
par rapport au poids total des alcanes dans ledit mélange.

Selon un mode de réalisation particulier, le mélange d'alcanes est un mélange n-undécane/n-tridécane. En particulier, un tel mélange peut être obtenu selon l'exemple 1 ou l'exemple 2 de la demande WO 2008/155059.

Selon un autre mode de réalisation particulier, on utilise le n-dodécane vendu sous la référence PARAFOL 12-97 par SASOL.

Selon un autre mode de réalisation particulier, on utilise le n-tétradécane vendu sous la référence PARAFOL 14-97 par SASOL.

Selon encore un autre mode de réalisation, on utilise un mélange de n-dodécane et de n-tétradécane.

La composition de l'invention peut comprendre de 0,5 % à 90 % en poids , en particulier de 1 % à 50 % en poids, plus particulièrement de 3 % à 40 % en poids et mieux de 3 à 30% en poids d'alcanes linéaires volatils par rapport au poids total de la composition.

Par "polymère cationique", on entend tout polymère contenant des groupements cationiques et/ou des groupements ionisables en groupements cationiques.

Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A- 2 270 846, 2 383 660, 2 598 611, 2 470 596 et 2 519 863.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont une masse moléculaire moyenne en poids supérieure à 10⁵, de préférence supérieure à 10⁶ et mieux encore comprise entre 10⁶ et 10⁸. Les polymères cationiques sont de préférence non siliconés.

Les polymères cationiques utilisés dans la présente invention sont non-protéiques, c'est-à-dire qu'ils ne comportent aucun bloc protéique ou motif à base d'acide aminé ou de peptide. Comme polymère cationique non-protéique, on peut citer plus particulièrement les polymères du type polyamine, polyaminoamide et polyammonium quaternaire. Ce sont des produits connus.

Les polymères du type polyamine, polyaminoamide, polyammonium quaternaire, que l'on peut utiliser dans la composition de la présente invention, sont ceux décrits dans les brevets français n^{os} 2 505 348 et 2 542 997. Parmi ces polymères, on peut citer :
(1) les homopolymères ou copolymères dérivés d'esters ou d'amides acryliques ou méthacryliques et comportant au moins un des motifs de formules suivantes: 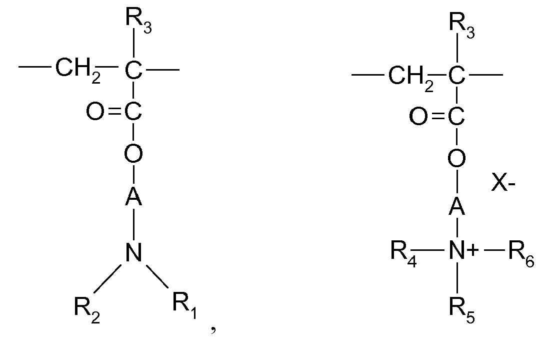 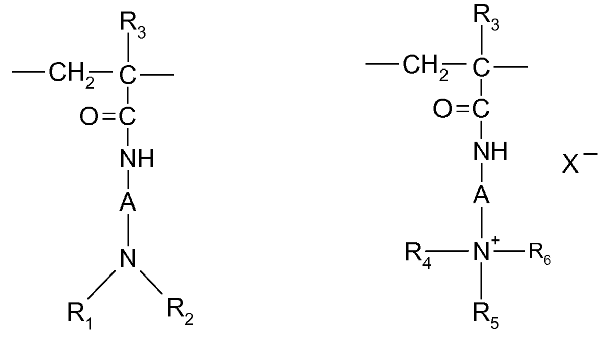 dans lesquelles:
   R₁ et R₂, identiques ou différents, représentent un atome d'hydrogène ou un groupe alkyle ayant de 1 à 6 atomes de carbone, et de préférence un groupe méthyle ou éthyle ;
   R₃, identiques ou différents, désignent un atome d'hydrogène ou un groupe CH₃ ;
   les symboles A, identiques ou différents, représentent un groupe alkyle, linéaire ou ramifié, comportant de 1 à 6 atomes de carbone, de préférence 2 ou 3 atomes de carbone, ou un groupe hydroxyalkyle comportant de 1 à 4 atomes de carbone ;
   R₄, R₅, R₆, identiques ou différents, représentent un groupe alkyle ayant de 1 à 18 atomes de carbone ou un groupe benzyle, et de préférence un groupe alkyle ayant de 1 à 6 atomes de carbone ;
   X désigne un anion dérivé d'un acide minéral ou organique tel qu'un anion méthosulfate ou un halogénure comme le chlorure ou le bromure.
   Les copolymères de la famille (1) peuvent contenir en outre un ou plusieurs motifs dérivant de comonomères pouvant être choisis dans la famille des acrylamides, méthacrylamides, diacétone-acrylamides, acrylamides et méthacrylamides substitués sur l'atome d'azote par des groupes alkyle inférieur (C₁-C₄), des groupes dérivés des acides acryliques ou méthacryliques ou de leurs esters, de vinyllactames tels que la vinylpyrrolidone ou le vinylcaprolactame, d'esters vinyliques.
   Ainsi, parmi ces copolymères de la famille (1), on peut citer :
   - les copolymères d'acrylamide et de méthacrylate de diméthylaminoéthyle quaternisé au sulfate de diméthyle ou avec un halogénure de méthyle,
   - les copolymères d'acrylamide et de chlorure de méthacryloyloxyéthyltriméthylammonium décrits, par exemple, dans la demande de brevet EP-A-080976,
   - les copolymères d'acrylamide et de méthosulfate de méthacryloyloxyéthyltriméthylammonium,
   - les copolymères vinylpyrrolidone/acrylate ou méthacrylate de dialkylaminoalkyle quaternisés ou non. Ces polymères sont décrits en détail dans les brevets français 2 077 143 et 2 393 573. Un tel polymère est commercialisé par exemple sous la dénomination commerciale GAFQUAT^{®} 755 par la société ISP,
   - les terpolymères méthacrylate de diméthylaminoéthyle/ vinylcaprolactame/vinylpyrrolidone,
   - les copolymères vinylpyrrolidone/méthacrylamidopropyl-diméthylamine,
   - les copolymères vinylpyrrolidone/méthacrylamide de diméthylaminopropyle quaternisé, et
   - les polymères réticulés de sels de méthacryloyloxyalkyl(C₁-C₄) trialkyl(C₁-C₄)ammonium tels que les polymères obtenus par homopolymérisation du méthacrylate de diméthylaminoéthyle quaternisé par le chlorure de méthyle, ou par copolymérisation de l'acrylamide avec le méthacrylate de diméthylaminoéthyle quaternisé par le chlorure de méthyle, l'homopolymérisation ou la copolymérisation étant suivie d'une réticulation par un composé à insaturation oléfinique, en particulier le méthylène-bisacrylamide.
      A titre de polymères réticulés de sels de méthacryloyloxyalkyl(C₁-C₄) trialkyl(C₁-C₄)ammonium on peut citer ceux commercialisés sous le nom de SALCARE^{®} SC 92, SALCARE^{®} SC 95 et SALCARE^{®} SC 96 par la Société CIBA;
(2) Les dérivés d'éthers de cellulose comportant des groupements ammonium quaternaires décrits dans le brevet français 1 492 597, et en particulier les polymères commercialisés sous les dénominations "UCARE POLYMER JR" (JR 400 LT, JR 125, JR 30M) ou " UCARE POLYMER LR" (LR 400, LR 30M) par la Société AMERCHOL. Ces polymères sont également définis dans le dictionnaire CTFA comme des ammoniums quaternaires d'hydroxyéthylcellulose ayant réagi avec un époxyde substitué par un groupement triméthylammonium.
(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkylcelluloses, comme les hydroxyméthyl-, hydroxyéthyl- ou hydroxypropyl-celluloses greffées notamment avec un sel de méthacryloyléthyl-triméthylammonium, de méthacrylamidopropyl-triméthylammonium, de diméthyldiallylammonium.
   Les produits commercialisés répondant à cette définition sont plus particulièrement les produits vendus sous la dénomination "Celquat^{®} L 200" et "Celquat^{®} H 100" par la Société National Starch.
(4) Les polysaccharides cationiques décrits dans les brevets US 3 589 578 et 4 031 307 tels que les gommes de guar contenant des groupements cationiques trialkylammonium. On utilise, par exemple, des gommes de guar modifiées par un sel, par exemple le chlorure, de 2,3-époxypropyltriméthylammonium.
   De tels produits sont commercialisés notamment sous les dénominations commerciales de JAGUAR^{®} C13 S, JAGUAR^{®} C15, JAGUAR^{®} C17 ou JAGUAR^{®} C162 par la société RHODIA.
(5) Les polymères constitués de motifs pipérazinyle et de groupes divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2 162 025 et 2 280 361.
(6) Les polyaminoamides solubles dans l'eau, préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bishaloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bishaloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alkylés ou s'ils comportent une ou plusieurs fonctions amines tertiaires, quaternisées. De tels polymères sont notamment décrits dans les brevets français 2 252 840 et 2 368 508.
(7) Les dérivés de polyaminoamides résultant de la condensation de polyalkylènes-polyamines avec des acides polycarboxyliques, suivie d'une alkylation par des agents bifonctionnels. On peut citer, par exemple, les polymères acide adipique/diakylaminohydroxyalkyl-dialkylènetriamine dans lesquels le groupe alkyle comporte de 1 à 4 atomes de carbone et désigne de préférence un groupe méthyle, éthyle, propyle, et le groupe alkylène comporte de 1 à 4 atomes de carbone, et désigne de préférence le groupe éthylène. De tels polymères sont notamment décrits dans le brevet français 1 583 363.
   Parmi ces dérivés, on peut citer plus particulièrement les polymères acide adipique/diméthylaminohydroxypropyl-diéthylènetriamine.
(8) Les polymères obtenus par réaction d'une polyalkylène-polyamine comportant deux groupements amine primaire et au moins un groupement amine secondaire, avec un acide dicarboxylique choisi parmi l'acide diglycolique et les acides dicarboxyliques aliphatiques saturés ayant de 3 à 8 atomes de carbone. Le rapport molaire entre la polyalkylène-polylamine et l'acide dicarboxylique étant compris entre 0,8 : 1 et 1,4 : 1 ; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3 227 615 et 2 961 347.
(9) Les cyclopolymères d'alkyldiallylamine ou de dialkyldiallylammonium tels que les homopolymères ou copolymères comportant, comme constituant principal de la chaîne, des motifs répondant aux formules (Ia) ou (Ib) : 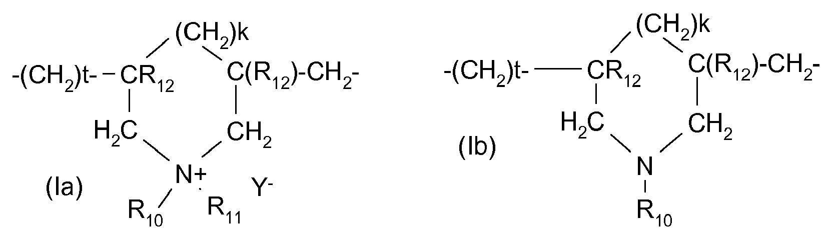 dans lesquelles k et t sont égaux à 0 ou 1, la somme k + t étant égale à 1 ; R₁₂ désigne un atome d'hydrogène ou un groupe méthyle ; R₁₀ et R₁₁, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C₁-C₄), ou alors R₁₀ et R₁₁ peuvent désigner conjointement avec l'atome d'azote auquel ils sont rattachés, des groupements hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2 080 759 et dans son certificat d'addition 2 190 406.
   R₁₀ et R₁₁, indépendamment l'un de l'autre, désignent de préférence un groupement alkyle ayant de 1 à 4 atomes de carbone.
   Parmi les polymères définis ci-dessus, on peut citer plus particulièrement l'homopolymère de chlorure de diméthyldiallylammonium vendu sous la dénomination "MERQUAT^{®} 100" par la société NALCO COMPANY (et ses homologues de faibles masses moléculaires moyenne en poids) et les copolymères de chlorure de diallyldiméthylammonium et d'acrylamide commercialisés sous la dénomination "MERQUAT^{®} 550" ou "MERQUAT^{®} 7SPR".
(10) Les polymères de diammonium quaternaire contenant des motifs récurrents répondant à la formule (II) : 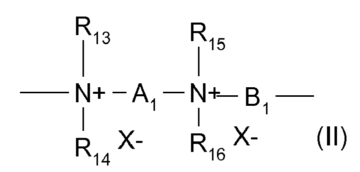 dans laquelle :
   R_{13,} R_{14,} R₁₅ et R₁₆, identiques ou différents, représentent des groupes aliphatiques, alicycliques ou arylaliphatiques contenant de 1 à 20 atomes de carbone ou des groupes hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote, ou bien R₁₃, R₁₄, R₁₅ et R₁₆ représentent un groupe alkyle en C₁-C₆, linéaire ou ramifié, substitué par un groupement nitrile, ester, acyle, amide ou -CO-O-R₁₇-E ou -CO-NH-R₁₇-E où R₁₇ est un groupe alkylène et E un groupement ammonium quaternaire ;
   A₁ et B₁ représentent des groupements polyméthyléniques contenant de 2 à 20 atomes de carbone, pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A₁, R₁₃ et R₁₅ peuvent former avec les deux atomes d'azote auxquels ils sont rattachés un cycle pipérazinique ; en outre, si A₁ désigne un groupe alkylène ou hydroxyalkylène linéaire ou ramifié, saturé ou insaturé, B₁ peut également désigner un groupement :

      -(CH₂)ₙ-CO-E'-OC-(CH₂)n-

      dans lequel E' désigne :
      a) un reste de glycol de formule -O-Z-O-, où Z désigne un groupe hydrocarboné linéaire ou ramifié, ou un groupement répondant à l'une des formules suivantes :

         -(CH₂-CH₂-O)ₓ-CH₂-CH₂-

         -[CH₂-CH(CH₃)-O]_{y}-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule -NH-Y-NH-, où Y désigne un groupe hydrocarboné linéaire ou ramifié, ou bien le groupe divalent -CH₂-CH₂-S-S-CH₂-CH₂- ;
      d) un groupement uréylène de formule -NH-CO-NH- .
   De préférence, X⁻ est un anion tel que le chlorure ou le bromure.
   Des polymères de ce type sont notamment décrits dans les brevets français 2 320 330, 2 270 846, 2 316 271, 2 336 434 et 2 413 907 et les brevets US 2 273 780, 2 375 853, 2 388 614, 2 454 547, 3 206 462, 2 261 002, 2 271 378, 3 874 870, 4 001 432, 3 929 990, 3 966 904, 4 005 193, 4 025 617, 4 025 627, 4 025 653, 4 026 945 et 4 027 020.
   On peut utiliser plus particulièrement les polymères qui sont constitués de motifs récurrents répondant à la formule (III): 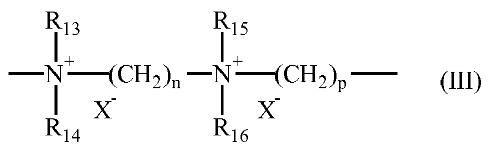 dans laquelle R₁₃, R₁₄, R₁₅ et R₁₆, identiques ou différents, désignent un groupe alkyle ou hydroxyalkyle ayant de 1 à 4 atomes de carbone environ, n et p sont des nombres entiers variant de 2 à 20 environ et, X⁻ est un anion dérivé d'un acide minéral ou organique.
(11) Les polymères de polyammonium quaternaire constitués de motifs de formule (IV) : 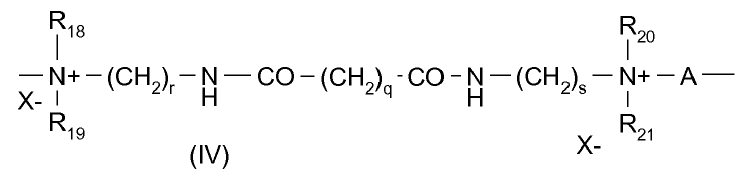 dans laquelle :
   R_{18,} R_{19,} R₂₀ et R_{21,} identiques ou différents, représentent un atome d'hydrogène ou un groupe méthyle, éthyle, propyle, β-hydroxyéthyle, β-hydroxypropyle ou -CH₂CH₂(OCH₂CH₂)pOH, où p est égal à 0 ou à un nombre entier compris entre 1 et 6, sous réserve que R₁₈, R₁₉, R₂₀ et R₂₁ ne représentent pas simultanément un atome d'hydrogène,
   r et s, identiques ou différents, sont des nombres entiers compris entre 1 et 6,
   q est égal à 0 ou à un nombre entier compris entre 1 et 34,
   X⁻ désigne un anion tel qu'un halogénure,
   A désigne un radical d'un dihalogénure ou représente de préférence -CH₂-CH₂-O-CH₂-CH₂-.
   De tels composés sont notamment décrits dans la demande de brevet EP-A-122 324.
(12) Les polymères quaternaires de vinylpyrrolidone et de vinylimidazole tels que, par exemple, les produits commercialisés sous les dénominations Luviquat^{®} FC 905, FC 550 et FC 370 par la société B.A.S.F.
   et leurs mélanges.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre ceux des familles (1), (2) (9) et (12) et plus particulièrement les copolymères vinylpyrrolidone/acrylate ou méthacrylate de dialkylaminoalkyle quaternisés tels que celui vendu sous la dénomination GAFQUAT^{®} 755 par la société ISP ; les dérivés d'éther de cellulose comportant des groupements ammonium quaternaires tels que les produits vendus sous la dénomination "UCARE POLYMER JR 400 LT" par la Société AMERCHOL ; les cyclopolymères cationiques, en particulier les homopolymères ou copolymères de chlorure de diméthyldiallylammonium, vendus sous les dénominations MERQUAT^{®} 100, MERQUAT^{®} 550, MERQUAT^{®} S et MERQUAT^{®} 7SPR par la société NALCO COMPANY ; les polymères quaternaires de vinylpyrrolidone et de vinylimidazole tels que, par exemple, les produits commercialisés sous les dénominations Luviquat^{®} FC 905, FC 550 et FC 370 par la société B.A.S.F ; les polymères réticulés de sels de méthacryloyloxyalkyl(C₁-C₄) trialkyl(C₁-C₄)ammonium tels que ceux commercialisés sous le nom de SALCARE^{®} SC 92, SALCARE^{®} SC 95 et SALCARE^{®} SC 96 par la Société CIBA ; et leurs mélanges.

Les polymères cationiques non-protéiques sont de préférence présents en une quantité allant de 0,05 à 10 % en poids, mieux encore de 0,1 à 5 % en poids, et encore plus préférentiellement de 0,2 à 2 % en poids par rapport au poids total de la composition.

Le milieu cosmétiquement acceptable est constitué de préférence d'eau ou d'un mélange d'eau et d'au moins un solvant cosmétiquement acceptable choisi parmi les alcools inférieurs en C₁-C₄, tels que l'éthanol, l'isopropanol, le tertio-butanol ou le n-butanol ; les polyols tels que le glycérol, le propylèneglycol et les polyéthylèneglycols ; et leurs mélanges.

Selon un mode de réalisation, la composition selon l'invention peut comprendre en outre au moins un alcool gras saturé ou insaturé, linéaire ou ramifié, comportant de 8 à 32 atomes de carbone, mieux encore de 10 à 22 atomes de carbone. A titre d'exemples d'alcools gras pouvant être utilisés dans la présente invention, on peut notamment citer les alcools laurique, myristique, cétylique, stéarique, cétylstéarylique, oléique et béhénylique, l'hexyldécanol, le 2-octyldodécanol, et leurs mélanges.

Lorsque l'alcool ou les alcool(s) gras sont présent(s), la composition selon l'invention peut en comprendre de 0,1 à 20 % en poids, en particulier de 0,2 à 15 %, et plus particulièrement de 0,5 à 10 % en poids par rapport au poids total de la composition.

De préférence, la composition selon l'invention peut comprendre en outre au moins un ester gras et/ou au moins une silicone, de préférence non-volatile.

Dans un mode de réalisation, la composition selon l'invention peut comprendre en outre au moins un ester gras d'un acide mono ou polycarboxylique en C2-30 et d'un mono ou polyalcool en C1-30, de préférence en C3-20, le nombre total d'atomes de carbone de l'ester variant de 10 à 50. En particulier, l'acide carboxylique et l'alcool de l'ester gras sont linéaires.

Comme exemples d'esters gras pouvant être utilisés dans la présente invention, on peut notamment citer le caprate/caprylate de 2-éthylhexyle (ou caprate/caprylate d'octyle), le laurate d'éthyle, le laurate de butyle, le laurate d'hexyle, le laurate d'isohexyle, le laurate d'isopropyle, le myristate de méthyle, le myristate d'éthyle, le myristate de butyle, le myristate d'isobutyle, le myristate d'isopropyle, le myristate de 2-octyldodécyle, le myristate de myristyle, le myristate de cétyle, le myristate de stéaryle, le monococoate de 2-éthylhexyle (ou monococoate d'octyle), le palmitate de méthyle, le palmitate d'éthyle, le palmitate d'isopropyle, le palmitate d'isobutyle, le palmitate de 2-éthylhexyle (ou palmitate d'octyle), le palmitate de myristyle, le palmitate de cétyle, le palmitate de stéaryle, le stéarate de butyle, le stéarate d'isopropyle, le stéarate d'isobutyle, le stéarate d'isocétyle, le stéarate de myristyle, le stéarate de cétyle, le stéarate de stéaryle, l'isostéarate d'isotéaryle, l'isostéarate d'isopropyle, le stéarate de 2-éthylhexyle (ou stéarate d'octyle), le pelargonate de 2-éthylhexyle (ou pelargonate d'octyle), l'hydroxystéarate de 2-éthylhexyle (ou hydroxystéarate d'octyle), l'oléate de décyle, l'adipate de di-isopropyle, l'adipate de di-2-éthylhexyle (ou adipate de di-octyle), l'adipate de diisocetyle, le succinate de 2-éthylhexyle (ou succinate d'octyle), le sébacate de diisopropyle, le malate de 2-éthylhexyle (ou malate d'octyle), le caprate/caprylate de pentaérythritol, le pentaérythritol de tétraisostéarate, l'hexanoate de 2-éthylhexyle (ou hexanoate d'octyle), l'octanoate d'octyldodécyle, le néopentanoate d'isodécyle, le néopentanoate d'isostéaryle, l'isononanoate de cétéaryle, l'isononanoate d'isodécyle, l'isononanoate d'isononyle, l'isononanoate d'isotridécyle, le lactate de lauryle, le lactate de myristyle, le lactate de cétyle, le propionate de myristyle, le 2-éthylhexanoate de 2-éthylhexyle (ou 2-éthylhexanoate d'octyle), l'octanoate de 2-éthylhexyle (ou octanoate d'octyle), les benzoates d'alcools gras en C12-C15 (Finsolv TN de FINETEX), le lauroyl sarcosinate d'isopropyle (Eldew SL 205 de Unipex), le dicaprylyl carbonate (Cetiol CC de Cognis) et leurs mélanges.

De préférence, l'ester gras pourra être choisi parmi le myristate de myristyle, le myristate de cétyle, le myristate de stéaryle, le palmitate de myristyle, le palmitate de cétyle, le palmitate de stéaryle, le stéarate de myristyle, le stéarate de cétyle, le stéarate de stéaryle, et leurs mélanges.

Lorsque l'ester ou les esters gras est ou sont présent(s), la composition selon l'invention peut en comprendre de 0,1 à 20 % en poids, en particulier de 0,5 à 15 %, et plus particulièrement de 1 à 10 % en poids par rapport au poids total de la composition.

La composition selon l'invention peut comprendre en outre au moins un autre corps gras solide comme des cires autres que les alcools gras ou les esters définis précédemment.

Par cire, on entend au sens de la présente invention, un composé lipophile, solide à température ambiante (25 °C), à changement d'état solide/liquide réversible, ayant un point de fusion supérieur ou égal à 30 °C, pouvant aller jusqu'à 200 °C et notamment jusqu'à 120 °C.

En portant une cire à l'état liquide (fusion), il est possible de la rendre miscible aux huiles et de former un mélange homogène macroscopiquement, mais en ramenant la température du mélange à la température ambiante, on obtient une recristallisation de la cire dans les huiles du mélange.

Une cire convenant à l'invention peut présenter un point de fusion supérieur ou égal à 45 °C, et en particulier, supérieur ou égal à 55 °C.

Au sens de l'invention, la température de fusion correspond à la température du pic le plus endothermique observé en analyse thermique (DSC), telle que décrite dans la norme ISO 11357-3 ; 1999. Le point de fusion de la cire peut être mesuré à l'aide d'un calorimètre à balayage différentiel (DSC), par exemple, le calorimètre vendu sous la dénomination « MDSC 2920 » par la société TA Instruments.

En particulier, une cire convenant à l'invention peut être choisie parmi des cires d'origine animale, végétale, minérale, synthétique, et leurs mélanges.

A titre illustratif de cires convenant à l'invention, on peut notamment citer les cires hydrocarbonées, comme la cire d'abeille, notamment d'origine biologique, la cire de lanoline, et les cires d'insectes de Chine; la cire de son de riz, la cire de Carnauba, la cire de Candellila, la cire d'Ouricury, la cire d'Alfa, la cire de Berry, la cire de Shellac, la cire du Japon et la cire de sumac; la cire de Montan, les cires d'orange et de citron, les cires microcristallines, les paraffines et l'ozokérite; les cires de polyéthylène, les cires obtenues par la synthèse de Fisher-Tropsch et les copolymères cireux, ainsi que leurs esters.

On peut également citer les cires microcristallines en C20-C60, telles que la Microwax HW.

On peut également citer la cire de polyéthylène PM 500 commercialisée sous la référence Permalen 50-L polyéthylène.

On peut aussi citer des cires obtenues par hydrogénation catalytique d'huiles animales ou végétales ayant des chaînes grasses, linéaires ou ramifiées, en C8-C32.

Parmi celles-ci, on peut notamment citer l'huile de jojoba isomérisée, telle que l'huile de jojoba partiellement hydrogénée isomérisée trans fabriquée ou commercialisée par la société Desert Whale sous la référence commerciale Iso-Jojoba-50®, l'huile de tournesol hydrogénée, l'huile de ricin hydrogénée, l'huile de coprah hydrogénée, l'huile de lanoline hydrogénée, et le tétrastéarate de di-(triméthylol-1,1,1 propane) vendu sous la dénomination de Hest 2T-4S® par la société HETERENE.

On peut encore citer les cires fluorées.

On peut également utiliser les cires obtenues par hydrogénation d'huile de ricin estérifiée avec l'alcool cétylique vendues sous les dénominations de Phytowax ricin 16L64® et 22L73® par la société SOPHIM. De telles cires sont décrites dans la demande FR-A- 2 792 190.

Comme cire, on peut utiliser un (hydroxystéaryloxy)stéarate d'alkyle en C20-C40 (le groupe alkyle comprenant de 20 à 40 atomes de carbone), seul ou en mélange.

Une telle cire est notamment vendue sous les dénominations « Kester Wax K 82 P® », « Hydroxypolyester K 82 P® » et « Kester Wax K 80 P® » par la société KOSTER KEUNEN.

Comme microcires pouvant être utilisées dans une composition de l'invention, on peut citer notamment les microcires de carnauba, telles que celle commercialisée sous la dénomination de MicroCare 350® par la société MICRO POWDERS, les microcires de cire synthétique, telles que celle commercialisée sous la dénomination de MicroEase 114S® par la société MICRO POWDERS, les microcires constituées d'un mélange de cire de carnauba et de cire de polyéthylène, telles que celles commercialisées sous les dénominations de Micro Care 300® et 310® par la société MICRO POWDERS, les microcires constituées d'un mélange de cire de carnauba et de cire synthétique, telles que celle commercialisée sous la dénomination Micro Care 325® par la société MICRO POWDERS, les microcires de polyéthylène, telles que celles commercialisées sous les dénominations de Micropoly 200®, 220®, 220L® et 2505® par la société MICRO POWDERS et les microcires de polytétrafluoroéthylène, telles que celles commercialisées sous les dénominations de Microslip 519® et 519 L® par la société MICRO POWDERS.

Selon un mode de réalisation, une cire convenant à l'invention peut, notamment, être choisie parmi la cire de Candellila, la cire de Carnauba, la cire de son de riz, la cire d'abeilles, notamment la cire d'abeille certifiée biologique, l'huile de jojoba isomérisée, et leurs mélanges.

Lorsqu'elle(s) est ou sont présente(s), la composition selon l'invention peut comprendre de 0,1 à 20 % en poids de cire(s), en particulier de 0,2 à 10 %, plus particulièrement de 0,5 à 5 %, et mieux encore de 0,5 % à 3 % en poids de cire(s) par rapport au poids total de la composition.

Dans un mode de réalisation, la composition selon l'invention peut comprendre en outre au moins une silicone.

Elle peut être volatile ou non-volatile, et de préférence non-volatile.

Les silicones utilisables conformément à l'invention peuvent être solubles ou insolubles dans la composition. Elles peuvent être en particulier des polyorganosiloxanes insolubles dans la composition de l'invention et se présenter sous forme d'huiles, de cires, de résines ou de gommes.

Les silicones insolubles sont notamment dispersées dans les compositions sous forme de particules ayant généralement une taille moyenne en nombre comprise entre 2 nanomètres et 100 micromètres, de préférence entre 20 nanomètres et 20 micromètres (mesurée avec un granulomètre).

Les organopolysiloxanes sont définis plus en détail dans l'ouvrage de Walter NOLL "Chemistry and Technology of Silicones" (1968) Academic Press. Elles peuvent être volatiles ou non volatiles, de préférence non-volatiles.

Lorsqu'elles sont volatiles, les silicones sont plus particulièrement choisies parmi celles possédant un point d'ébullition compris entre 60° C et 260° C, et plus particulièrement encore parmi :
(i) les silicones cycliques comportant de 3 à 7 atomes de silicium et, de préférence, 4 à 5. Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHODIA, le décaméthylcyclopentasiloxane commercialisé sous le nom de "VOLATILE SILICONE 7158" par UNION CARBIDE, "SILBIONE 70045 V 5" par RHODIA, ainsi que leurs mélanges.
   On peut également citer les cyclocopolymères du type diméthylsiloxane/méthylalkylsiloxane, tel que la "SILICONE VOLATILE FZ 3109" commercialisée par la société UNION CARBIDE, de structure chimique : 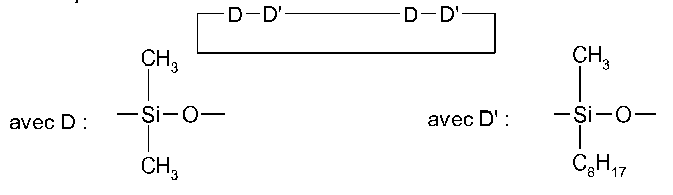 On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25 °C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, p. 27-32 - TODD & BYERS "Volatile Silicone fluids for cosmetics".

Parmi les silicones non volatiles, on peut notamment citer les polyalkylsiloxanes, les polyarylsiloxanes, les polyalkylarylsiloxanes, les gommes et les résines de silicones, les polyorganosiloxanes modifiés par des groupements organofonctionnels, les copolymères blocs linéaires polysiloxane(A)-polyoxyalkylène(B) de type (A-B)ₙ avec n >3 ; les polymères siliconés greffés, à squelette organique non siliconé, constitués d'une chaîne principale organique formée à partir de monomères organiques ne comportant pas de silicone, sur laquelle se trouve greffé, à l'intérieur de ladite chaîne ainsi qu'éventuellement à l'une au moins de ses extrémités, au moins un macromonomère polysiloxane ; les polymères siliconés greffés, à squelette polysiloxanique greffé par des monomères organiques non siliconés, comprenant une chaîne principale de polysiloxane sur laquelle se trouve greffé, à l'intérieur de ladite chaîne ainsi qu'éventuellement à l'une au moins de ses extrémités, au moins un macromonomère organique ne comportant pas de silicone ; ainsi que leurs mélanges.

A titre d'exemples de polyalkylsiloxanes, on peut notamment citer les polydiméthylsiloxanes à groupements terminaux triméthylsilyle ayant une viscosité de 5.10⁻⁶ à 2,5 m²/s à 25°C et de préférence 1.10⁻⁵ à 1 m²/s. La viscosité des silicones est par exemple mesurée à 25°C selon la norme ASTM 445 Appendice C.

Parmi ces polyalkylsiloxanes, on peut citer à titre non limitatif les produits commerciaux suivants :
- les huiles SILBIONE des séries 47 et 70 047 ou les huiles MIRASIL commercialisées par RHONE POULENC telles que par exemple l'huile 70 047 V 500 000 ;
- les huiles de la série MIRASIL commercialisées par la société RHONE POULENC ;
- les huiles de la série 200 de la société DOW CORNING telles que plus particulièrement la DC200 de viscosité 60 000 cSt ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHONE POULENC.

Dans cette classe de polyalkylsiloxanes, on peut également citer les produits commercialisés sous les dénominations "ABIL WAX 9800 et 9801" par la société GOLDSCHMIDT qui sont des polyalkyl (C₁-C₂₀) siloxanes.

Les polyalkylarylsiloxanes peuvent être notamment choisis parmi les polydiméthyl-méthylphénylsiloxanes, les polydiméthyl-diphényl-siloxanes linéaires et/ou ramifiés de viscosité de 1.10⁻⁵ à 5.10⁻² m²/s à 25°C.

Parmi ces polyalkylarylsiloxanes, on peut citer à titre d'exemple les produits commercialisés sous les dénominations suivantes :
- les huiles SILBIONE de la série 70 641 de RHONE POULENC ;
- les huiles des séries RHODORSIL 70 633 et 763 de RHONE POULENC ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Les gommes de silicone utilisables conformément à l'invention sont notamment des polydiorganosiloxanes ayant des masses moléculaires moyennes en nombre élevées comprises entre 200 000 et 1 000 000, utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- les gommes polydiméthylsiloxane,
- les gommes polydiméthylsiloxanes/méthylvinylsiloxane,
- les gommes polydiméthylsiloxane/diphénylsiloxane,
- les gommes polydiméthylsiloxane/phénylméthylsiloxane,
- les gommes polydiméthylsiloxane / diphénylsiloxane / méthylvinylsiloxane.

Des silicones que l'on peut utiliser dans la composition selon l'invention sont des mélanges tels que :
- les mélanges formés à partir d'un polydiméthylsiloxane hydroxylé en bout de chaîne (dénommé diméthiconol selon la nomenclature du dictionnaire CTFA) et d'un polydiméthylsiloxane cyclique (dénommé cyclométhicone selon la nomenclature du dictionnaire CTFA) tel que le produit Q2 1401 commercialisé par la société DOW CORNING ;
- les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 20 et 85 % d'une huile SF 96.

Les résines d'organopolysiloxanes utilisables conformément à l'invention sont des systèmes siloxaniques réticulés renfermant les unités : R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle.

Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone-copolyol commercialisé par la société DOW CORNING sous la dénomination DC 1248 ou les huiles SILWET^{®} L 722, L 7500, L 77, L 711 de la société UNION CARBIDE et l'alkyl(C₁₂)-méthicone-copolyol commercialisée par la société DOW CORNING sous la dénomination Q2 5200 ;
- des groupements aminés substitués ou non, comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;
- des groupements ammonium quaternaires comme les produits commercialisés sous les dénominations ABILQUAT 3272 et ABILQUAT 3474 par la société GOLDSCHMIDT ;

- des groupements thiols, comme les produits commercialisés sous les dénominations "GP 72 A" et "GP 71" de GENESEE ;
- des groupements alcoxylés, comme le produit commercialisé sous la dénomination "SILICONE COPOLYMER F-755" par SWS SILICONES et ABIL WAX^{®} 2428, 2434 et 2440 par la société GOLDSCHMIDT ;
- des groupements hydroxylés, comme les polyorganosiloxanes à fonction hydroxyalkyle décrits dans la demande de brevet français FR-A-85 16334 ;
- des groupements acyloxyalkyle tels que, par exemple, les polyorganosiloxanes décrits dans le brevet US-A-4957732.
- des groupements anioniques du type acide carboxylique comme, par exemple, dans les produits décrits dans le brevet EP 186 507 de la société CHISSO CORPORATION, ou du type alkyl-carboxylique comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations "ABIL^{®} S201" et "ABIL^{®} S255".
- des groupements hydroxyacylamino, comme les polyorganosiloxanes décrits dans la demande EP 342 834. On peut citer, par exemple, le produit Q2-8413 de la société DOW CORNING.

Les silicones particulièrement préférées dans l'invention sont les polydiméthylsiloxanes à fonctions terminales triméthylsilyles ou diméthylsilanol, les polydiméthylsiloxanes à fonctions amines, et leurs mélanges.

Dans un mode de réalisation particulier, la silicone est un polydiméthylsiloxane à fonctions terminales triméthylsilyle tel que celui vendu sous la dénomination commerciale BELSIL DM 300 000 par la société Wacker.

Lorsqu'au moins une desdites silicones est présente, elle(s) est ou sont contenue(s) de préférence en une quantité allant de 0,1 à 20 % en poids, plus particulièrement de 0,2 à 10 % en poids, mieux de 0,5 à 5 %, et mieux encore de 0,5 à 4% en poids par rapport au poids total de la composition.

La composition selon l'invention peut comprendre en outre un ou plusieurs additifs classiques bien connus dans la technique, tels que des polymères associatifs ou non, ioniques ou non-ioniques, des polyols, des protéines, des vitamines, des agents réducteurs, des plastifiants, des adoucissants, des agents anti-mousse, des agents hydratants, des pigments, des argiles, des charges minérales, des filtres UV, des colloïdes minéraux, des peptisants, des solubilisants, des parfums, des conservateurs, des tensio-actifs anioniques, cationiques, non ioniques ou amphotères, des agents nacrants, des propulseurs, et des épaississants minéraux ou organiques, des agents antipelliculaires, des agents antigras, des agents anti chute ou pour la repousse des cheveux.

L'homme de métier veillera à choisir les éventuels additifs et leur quantité de manière à ce qu'ils ne nuisent pas aux propriétés des compositions de la présente invention.

Ces additifs sont généralement présents dans la composition selon l'invention en une quantité allant de 0 à 20 % en poids par rapport au poids total de la composition.

Les compositions selon l'invention peuvent se présenter sous la forme de compositions de soin rincé ou non rincé, ces dernières se présentant sous la forme d'une lotion plus ou moins épaissie, d'une crème, d'un gel ou d'une émulsion.

Un autre objet de l'invention est l'utilisation de la composition cosmétique telle que décrite ci-dessus pour le traitement cosmétique des matières kératiniques, de préférence des fibres kératiniques comme les cheveux.

L'invention est également relative à un procédé de traitement cosmétique des matières kératiniques, de préférence des fibres kératiniques comme les cheveux, qui consiste à appliquer une quantité efficace d'une composition cosmétique telle que décrite ci-dessus, sur lesdites matières, à éventuellement rincer après un éventuel temps de pause.

Lorsque l'on applique la composition selon l'invention sous forme d'une lotion ou d'une crème, on la laisse éventuellement pauser sur les cheveux pendant environ 1/2 min. à 5 minutes, puis on rince éventuellement à l'eau.

Les exemples suivants sont donnés à titre illustratif de la présente invention.

Dans les exemples suivants, toutes les quantités sont indiquées en pour cent en poids de produit tel quel par rapport au poids total de la composition, sauf indication contraire.

### EXEMPLES

### Exemple 1

On a préparé la composition de soin rincé suivante à partir des ingrédients indiqués dans le tableau ci-dessous.

| | |
|---|---|
| Mélange majoritairement composé de n-undécane et de n-tridécane selon l'exemple 2 de WO 2008/155059 | 8,5 % |
| Homopolymère chlorure de méthacrylate d'éthyl-triméthylammonium réticulé, en émulsion inverse à 50% MA dans une huile minérale, vendu sous la dénomination commerciale Salcare SC 95 par la société CIBA | 1 % |
| Alcool cétylstéarylique (C16/C18/ 50/50) vendu sous la dénomination commerciale Lanette O OR par la société COGNIS | 8 % |
| Mélange de myristate/palmitate/stéarate de myristyle/cétyle/stéaryle vendu sous la dénomination commerciale Crodamol MS-PA(MH) par la société CRODA | 1,5 % |
| Polydiméthylsiloxane vendu sous la dénomination commerciale Belsil DM 300000 par la société Wacker Chemie AG | 1,5 % |
| Cire de candélilla | 2 % |
| Distéarate de polyéthylène glycol (150 moles d'oxyde d'éthylène) vendu sous la dénomination commerciale KESSCO PEG 6000 DS par la société ITALMATCH CHEMICALS ARESE | 0,5 % |
| Phosphate de diamidon de maïs hydroxypropylé prégélatinisé en dispersion aquese à 88 % MA (STRUCTURE ZEA de AKZO NOBEL) | 0,2 % |
| Copolymère PEG-150/alcool stéarylique/SMDI à 15 % en poids dans une matrice maltodextrine/eau vendu sous la dénomination commerciale Aculyn 46 par la société Rohm & Haas | 3 % |
| Conservateur | qs |
| Parfum | qs |
| Eau qsp | 100 % |

| | |
|---|---|
| MA : Matière Active | |

Cette composition de soin rincé a été testée sur têtes et comparée à une composition comparative identique comprenant un solvant volatile siliconé, un cyclopentasiloxane, à la place du mélange d'undécane et de tridécane. On a appliqué à température ambiante ces compositions sur les cheveux pendant une minute et rincé ensuite.

On a observé une amélioration du lissage, de la douceur et de la souplesse avec la composition selon l'invention par rapport à la composition comparative.

On a observé également cette amélioration lorsque l'on a remplacé le mélange d'undécane et de tridécane, par le n-dodécane, le n-tétradécane ou le mélange de n-dodécane et de n-tétradécane dans la composition, par rapport à la composition comparative comprenant le cyclopentasiloxane.

### Exemple 2

On a préparé la composition de soin non-rincé suivante à partir des ingrédients indiqués dans le tableau ci-dessous.

| | |
|---|---|
| Mélange n-dodécane/n-tétradécane vendu sous la dénomination commerciale Vegelight 1214 par la société BIOSYNTHIS | 2 % |
| Homopolymère chlorure de méthacrylate d'éthyl-triméthylammonium réticulé, en dispersion dans un mélange d'esters à 50%, vendu sous la dénomination commerciale Salcare SC 96 par la société CIBA | 1 % |
| Polydiméthylsiloxane vendu sous la dénomination commerciale Belsil DM 300000 par la société Wacker Chemie AG | 2 % |
| Copolymère PEG-150/alcool stéarylique/SMDI à 15 % en poids dans une matrice maltodextrine/eau vendu sous la dénomination commerciale Aculyn 46 par la société Rohm & Haas | 2,5 % |
| Conservateurs | qs |
| Parfum | qs |
| Eau qsp | 100 % |

On a appliqué cette composition sur les cheveux. Après séchage, on a observé d'excellents résultats de lissage, douceur et souplesse.

### Exemple 3

On a préparé la composition de soin non-rincé suivante à partir des ingrédients indiqués dans le tableau ci-dessous.

| | |
|---|---|
| Mélange majoritairement composé de n-undécane/n-tridécane selon l'exemple 2 de WO 2008/155059 | 3 % |
| Copolymère vinylpyrrolidone/chlorire de méthylvinylimidazolium (70/30) en solution aqueuse à 40% MA vendu sous la dénomination commerciale Luviquat FC 370 par la société BASF | 2,5 % |
| Copolymère chlorure de diméthyl-diallylammonium/acrylamide (30/70 en mole) en solution aqueuse à 9,5% en poids vendu sous la dénomination commerciale Merquat 7SPR par la société NALCO COMPANY | 1 % |
| Polydiméthylsiloxane vendu sous la dénomination commerciale Belsil DM 300000 par la société Wacker Chemie AG | 3 % |
| 2-amino-2-méthyl-1-propanol | 0,55 % |
| Phényl triméthylsiloxytrisiloxane vendu sous la dénomination commerciale Dow Corning 556 Cosmetic Grade Fluid par la société Dow Corning | 0,5 % |
| Huile de ricin hydrogénée oxyéthylénée (40 moles d'oxyde d'éthyléne) | 0,5 % |
| Acrylates/C10-C30 acrylate crosspolymer vendu sous la dénomination commerciale Pemulen TR-1 Polymer par la société Lubrizol | 0,6 % |
| Hydroxyéthylcellulose quaternisée par du chlorure de 2,3-époxypropyl triméthylammonium vendu sous la dénomination commerciale Ucare Polymer JR 400 LT par la société AMERCHOL | 0,4 % |
| Conservateurs | qs |
| Parfum | qs |
| Eau qsp | 100 % |

| | |
|---|---|
| MA : Matière Active | |

On a appliqué cette composition sur les cheveux. Après séchage, on a observé un très bon lissage visuel, un excellent toucher et une très grande facilité de mise en forme.

Le lissage visuel et la facilité de mise en forme sont supérieurs à ceux obtenus avec une composition identique dans laquelle le mélange undécane/tridécane est remplacé par le cyclopentasiloxane.

### Exemple 4

On a préparé la composition de soin non-rincé suivante à partir des ingrédients indiqués dans le tableau ci-dessous.

| | |
|---|---|
| Mélange majoritairement composé de n-undécane/n-tridécane selon l'exemple 2 de WO 2008/155059 | 1 % |
| Copolymère vinylpyrrolidone/méthacrylate de diméthylaminoéthyle quaternisé par du sulfate de diéthyle en solution aqueuse à 20% MA vendu sous la dénomination commerciale Gafquat 755 par la société ISP | 1 % |
| Homopolymère chlorure de méthacrylate d'éthyl-triméthylammonium réticulé, en dispersion dans un mélange d'esters à 50%, vendu sous la dénomination commerciale Salcare SC 96 par la société CIBA | 1 % |
| Polydiméthylsiloxane à groupements aminoéthyliminopropyl, à fonctions méthoxy et/ou hydroxy et alpha-oméga-silanol en émulsion aqueuse cationique à 60 % vendu sous la dénomination commerciale Dow Corning 2-8299 Cationic emulsion par la société Dow Corning | 1 % |
| Huile de ricin hydrogénée oxyéthylénée (40 moles d'oxyde d'éthylène) | 1,5 % |
| Glycérol | 3 % |
| Octane-1,2-diol | 0,5 % |
| Conservateurs | qs |
| Parfum | qs |
| Eau qsp | 100 % |

| | |
|---|---|
| MA : Matière Active | |

On a appliqué cette composition sur les cheveux. Après séchage, on a observé un très bon lissage visuel, un excellent toucher et une très bonne facilité de mise en forme.

Le lissage visuel et la facilité de mise en forme sont supérieurs à ceux obtenus avec une composition identique dans laquelle le mélange undécane/tridécane est remplacé par le cyclopentasiloxane

Les compositions exemplifiées peuvent être pulvérisables, via par exemple un flacon pompe-spray.

La composition de l'exemple 4 a été conditionnée dans un flacon pompe-spray. On a pu alors répartir facilement la composition pulvérisée sur les cheveux.

## Revendications

1. Composition cosmétique comprenant dans un milieu cosmétiquement acceptable :
- deux ou plus de deux alcanes linéaires volatils, et
- un ou plusieurs polymères cationiques non-protéiques, en un rapport pondéral alcanes linéaires volatils/polymère(s) cationique(s) non-protéique(s) supérieur ou égal à 1,2.

2. Composition cosmétique selon la revendication 1, **caractérisée en ce que** les alcanes linéaires volatils sont des alcanes linéaires comportant de 7 à 15 atomes de carbone.

3. Composition cosmétique selon la revendication 1 ou 2, **caractérisée en ce que** les alcanes linéaires volatils sont choisis parmi le n-nonane, le n-undécane, le n-dodécane, le n-tridécane, le n-tétradécane.

4. Composition cosmétique selon l'une quelconque des revendications précédentes, **caractérisée en ce que** les alcanes linéaires volatils sont présents en une teneur de 0,5 % à 90 % en poids, en particulier de 1 % à 50 % en poids, plus particulièrement de 3 % à 40 % en poids et mieux de 3 à 30% en poids par rapport au poids total de la composition.

5. Composition cosmétique selon l'une quelconque des revendications précédentes, **caractérisée en ce que** le polymère cationique est choisi parmi les copolymères vinylpyrrolidone/acrylate ou méthacrylate de dialkylaminoalkyle quaternisés ; les dérivés d'éther de cellulose comportant des groupements ammonium quaternaires ; les cyclopolymères cationiques, en particulier les homopolymères ou copolymères de chlorure de diméthyldiallylammonium ; les polymères quaternaires de vinylpyrrolidone et de vinylimidazole ; les polymères réticulés de sels de méthacryloyloxyalkyl(C₁-C₄) trialkyl(C₁-C₄)ammonium ; et leurs mélanges:

6. Composition cosmétique selon l'une quelconque des revendications précédentes, **caractérisée en ce que** le ou les polymères cationiques non protéiques sont présents en une teneur allant de 0,05 à 10 % en poids, mieux encore de 0,1 à 5 % en poids, et encore plus préférentiellement de 0,2 à 2 % en poids par rapport au poids total de la composition.

7. Composition cosmétique selon l'une quelconque des revendications précédentes, caractérisée en ce le rapport pondéral alcanes linéaires volatils/polymère(s) cationique(s) non-protéique(s) va de 1,2 à 50, mieux de 2 à 30 et mieux encore de 3 à 20.

8. Composition cosmétique selon l'une quelconque des revendications précédentes, **caractérisée en ce qu**'elle comprend au moins un alcool gras saturé ou non saturé, linéaire ou ramifié, comportant de 8 à 32 atomes de carbone.

9. Composition cosmétique selon l'une quelconque des revendications précédentes, **caractérisée en ce qu**'elle comprend au moins un esters gras d'un acide mono ou polycarboxylique en C2-30 et d'un mono ou polyalcool en C1-30, de préférence en C3-20, le nombre total d'atomes de carbone de l'ester variant de 10 à 50.

10. Composition cosmétique selon la revendication 9, **caractérisée en ce que** le ou les esters gras sont choisis parmi le myristate de myristyle, le myristate de cétyle, le myristate de stéaryle, le palmitate de myristyle, le palmitate de cétyle, le palmitate de stéaryle, le stéarate de myristyle, le stéarate de cétyle, le stéarate de stéaryle, et leurs mélanges

11. Composition cosmétique selon l'une quelconque des revendications précédentes, **caractérisée en ce qu**'elle comprend en outre au moins une silicone, de préférence non volatile.

12. Utilisation de la composition cosmétique selon l'une quelconque des revendications précédentes, pour le traitement des matières kératiniques, de préférence des cheveux.

13. Procédé de traitement des matières kératiniques, de préférences des fibres kératiniques, et mieux encore des cheveux, comprenant l'application de la composition cosmétique selon l'une quelconque des revendications 1 à 11.
